(19) Europäisches Patentamt
European Patent Office
Office européen des brevets

(11) EP 3 403 574 A1

(12) EUROPEAN PATENT APPLICATION

(43) Date of publication:
21.11.2018 Bulletin 2018/47

(51) Int Cl.:
A61B 5/024 (2006.01)      A61B 5/00 (2006.01)
A61B 5/021 (2006.01)      A61B 5/08 (2006.01)
A61B 5/1455 (2006.01)

(21) Application number: 17171812.5

(22) Date of filing: 18.05.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Preventicus GmbH
07745 Jena (DE)

(72) Inventor: Hübner, Thomas
07749 Jena (DE)

(74) Representative: Sutto, Luca
PGA S.P.A., Milano
Succursale di Lugano
Via Castagnola, 21c
6900 Lugano (CH)

(54) DEVICE FOR RELIABLE ACQUISITION OF PHOTOPLETHYSMOGRAPHIC DATA

(57) An apparatus for determining a pulse wave signal representative of vital signs of a subject is disclosed. The apparatus comprises a control unit, a first sensor coupled to the control unit and configured for emitting a first signal indicative of a pulse wave of a subject, and a second sensor coupled to the control unit and configured for detecting motion the apparatus is subjected to and for emitting a second signal based on the detected motion. The control unit is configured to receive the first signal from the first sensor, to determine a pulse wave signal based on the first signal to receive the second signal from the second sensor, and to determine a reliability signal based on the second signal. The reliability signal is indicative of a reliability of the first signal.

FIG.1A

**Description**

**Technical Field**

The present invention relates to reliable acquisition of photoplethysmographic data representative of vital signals of a subject. The processing includes determining whether a recorded pulse wave fulfills pre-determined quality requirements. Based on subsequent pulse waveform analysis, data pertaining to, for example, the heart rhythm, heart rate, respiratory rate, and/or blood pressure of a human subject can be determined and processed.

**Background Art**

A photoplethysmogram (PPG) is an optically obtained plethysmogram, a volumetric measurement of an organ. A PPG may be obtained by using a pulse oximeter, which illuminates the skin or other tissue of a subject and measures changes in light absorption. A conventional pulse oximeter typically monitors the perfusion of blood to the dermis and subcutaneous tissue of the skin. Pulse wave data or a pulse wave signal indicative of vital signals of a subject are/is regarded as representing a photoplethysmogram.

With each cardiac cycle the heart pumps blood to the periphery. Even though the corresponding pressure pulses are somewhat attenuated travelling from the heart through the vascular system and towards an organ, for example the skin of a human subject, the residual pressure pulses are sufficiently strong in order to distend the arteries and arterioles in the subcutaneous tissue.

The change in volume caused by the pressure pulse can be detected by illuminating the skin with the light from a light-emitting diode (LED) and by measuring the amount of light either transmitted or reflected to a photodiode. Each cardiac cycle appears as a peak, as seen in the figure. Because blood flow to the skin can be modulated by multiple other physiological systems, the PPG can also be used to monitor breathing, hypovolemia, and other circulatory conditions. Additionally, the shape of the PPG waveform differs from subject to subject, and varies with the location and manner in which the pulse oximeter is attached.

"Photoplethysmogram signal quality estimation using repeated Gaussian filters and cross-correlation", W. Karlen, K. Kobayashi, J. M. Ansermino, and G. A. Dumont, Physiol. Meas. 33 (2012) 1617-1629, discloses that pulse oximeters, i.e. monitors that noninvasively measure heart rate and blood oxygen saturation (SpO2), are typically prone to artifacts which negatively impact the accuracy of the measurement and can cause a significant number of false alarms. An algorithm is described, which segments pulse oximetry signals into pulses and estimates the signal quality in real time. The algorithm iteratively calculates a signal quality index (SQI) ranging from 0 to 100. In the presence of artifacts and irregular signal morphology, the algorithm outputs a low SQI number. The pulse segmentation algorithm uses the derivative of the signal to find pulse slopes and an adaptive set of repeated Gaussian filters to select the correct slopes. Cross-correlation of consecutive pulse segments is used to estimate signal quality. Experimental results using two different benchmark data sets showed a good pulse detection rate with a sensitivity of 96.21% and a positive predictive value of 99.22%, which was equivalent to the available reference algorithm. The SQI algorithm was effective and produced significantly lower SQI values in the presence of artifacts compared to SQI values during clean signals. The SQI algorithm may help to guide untrained pulse oximeter users and also help in the design of advanced algorithms for generating smart alarms.

"Optimal Signal Quality Index for Photoplethysmogram Signals", Mohamed Elgendi, Bioengineering 2016, 3, 21, discloses that photoplethysmogram (PPG) signals collected via mobile devices may be prone to artifacts that negatively impact measurement accuracy, which can lead to a significant number of misleading diagnoses and identifies developing an optimal signal quality index (SQI) as being essential for classifying the signal quality from such devices. Eight SQIs were developed and tested based on: perfusion, kurtosis, skewness, relative power, non-stationarity, zero crossing, entropy, and the matching of systolic wave detectors. Two independent annotators annotated all PPG data (106 recordings, 60 s each) and a third expert conducted the adjudication of differences. The independent annotators labeled each PPG signal with one of the following labels: excellent, acceptable or unfit for diagnosis. All indices were compared using Mahalanobis distance, linear discriminant analysis, quadratic discriminant analysis, and support vector machine with leave-one-out cross-validation. The skewness index outperformed the other seven indices in differentiating between excellent PPG and acceptable, acceptable combined with unfit, and unfit recordings, with overall F1 scores of 86.0%, 87.2%, and 79.1%, respectively.

An aim of the present invention is to provide an apparatus for reliably acquiring photoplethysmographic data representative of vital signals of a subject. The apparatus facilitates determining whether a recorded pulse wave fulfills pre-determined quality requirements such that pulse wave data not fulfilling the pre-determined quality requirements can be discarded or disregarded. From a recorded pulse wave, biological parameters of a subject may be determined, for example heart rate, respiration, blood pressure, and the variabilities thereof, in a noninvasive manner.

It is a further aim to provide an apparatus for reliably acquiring photoplethysmographic data representative of vital signals of a subject, and for determining biological parameters of a subject and the variabilities thereof with an improved accuracy.

It is a further aim to provide an apparatus for reliably acquiring photoplethysmographic data representative of vital signals of a subject, where the acquired photopletysmographic data are of improved quality. For example, the apparatus provides acquired photopletysmographic data substantially free from unwanted data acquisition artifacts, such as measurement artifacts. Such artifacts include inaccurate data or data compromised by measurement errors.

In particular, the apparatus is a mobile device, and preferably a conventional smart phone provided with a light source and an optical sensor.

## Summary of invention

According to the invention, in a 1st aspect there is provided an apparatus for determining a pulse wave signal representative of vital signs of a subject. The apparatus comprises a control unit, a first sensor coupled to the control unit and configured for emitting a first signal indicative of a pulse wave of a subject, and a second sensor coupled to the control unit and configured for detecting motion the apparatus is subjected to and for emitting a second signal based on the detected motion. The control unit is configured to receive the first signal from the first sensor, to determine a pulse wave signal based on the first signal, to receive the second signal from the second sensor, and to determine a reliability signal based on the second signal. The reliability signal is indicative of a reliability of the first signal.

In a 2nd aspect according to the 1st aspect, the reliability signal is further indicative of a reliability of the pulse wave signal. Optionally, the control unit (530) is further configured to determine one or more correlation values based on the pulse wave signal, and determining the reliability signal is further based on the one or more correlation values.

In a 3rd aspect according to the preceding aspect, the control unit is further configured to determine one or more perfusion indices based on the pulse wave signal, and determining the reliability signal is further based on the one or more perfusion indices.

In a 4th aspect according to any one of the two preceding aspects, the control unit is further configured to determine one or more frequency spectra based on the pulse wave signal, and determining the reliability signal is further based on the one or more frequency spectra.

In a 5th aspect according to any one of the preceding aspects, the control unit is further configured to determine a verified pulse wave signal based on the pulse wave signal and the reliability signal.

In a 6th aspect according to the preceding aspect, determining the verified pulse wave signal comprises one or more of selectively discarding one or more portions of the pulse wave signal based on the reliability signal and determining the verified pulse wave signal based on the pulse wave signal without the discarded one or more portions of the pulse wave signal, and selectively selecting one or more portions of the pulse wave signal based on the reliability signal and determining the verified pulse wave signal based on the selected one or more portions of the pulse wave signal.

In a 7th aspect according to aspect 5, determining the verified pulse wave signal comprises selectively assigning a signal quality index (SQI) to one or more portions of the pulse wave signal based on the reliability signal. Determining the verified pulse wave signal is exclusively based on the one or more portions of the pulse wave signal where each of the one or more portions of the pulse wave signal has assigned thereto a signal quality index fulfilling a minimum requirement.

In a n 8th aspect according to the preceding aspect, the signal quality index comprising one or more of one or more discrete values, the one or more discrete values optionally being selected from a predetermined set of discrete values, and a numeric value, the numeric value optionally falling within a predetermined numeric range ranging from a minimum value to a maximum value.

In a 9th aspect according to any one of the two preceding aspects, fulfilling a minimum requirement includes one or more of exceeding a minimum value, falling within a range defined by a minimum value and a maximum value, and not exceeding a maximum value.

In a 10th aspect according to any one of the preceding aspects, the second sensor is configured to detect one or more of a first acceleration along a first axis, a second acceleration along a second axis, a third acceleration along a third axis, a first rotation about the first axis, a second rotation about the second axis, and a third rotation about the third axis.

In an 11th aspect according to any one of the preceding aspects, determining the reliability signal is further based on the first, second, and/or third acceleration. Optionally, determining the reliability signal includes determining whether the first, second, and/or third acceleration exceeds a predetermined acceleration threshold value. Alternatively or additionally, determining the reliability signal is further based on the first, second, and/or third rotation. Optionally, determining the reliability signal includes determining whether the first, second, and/or third rotation exceeds a predetermined rotation threshold value.

In a 12th aspect according to any one of the preceding aspects, the apparatus further comprises a main body configured to carry the control unit, the first sensor, and the second sensor.

In a 13th aspect according to any one of the preceding aspects, the first sensor is configured for detecting light reflected from and/or permeating through tissue of the subject, and emitting the first signal is based on the detected light.

In a 14th aspect according to any one of the preceding aspects, the first sensor includes one or more of an optical sensor, a CCD sensor, a heart rate monitor (HRM).

In a 15th aspect according to any one of the preceding aspects, the apparatus further comprises a light source coupled to the control unit and configured to illuminate tissue of the subject. Optionally, the control unit is configured to control the light source to selectively illuminate tissue of the subject.

In a 16th aspect according to the preceding aspect, the light source is arranged in close proximity to the first sensor, and/or the light source is configured for illuminating tissue of the subject positioned in close proximity or in contact with the first sensor.

In a 17th aspect according to any one of the preceding aspects, the second sensor includes one or more of an accelerometer, a magnetometer, and a gyroscope.

In an 18th aspect according to any one of the preceding aspects, the control unit is configured to control the first sensor to emit the first signal, and/or control the second sensor to emit the second signal.

In a 19th aspect according to any one of the preceding aspects, the pulse wave signal is representative of a heart beat of the subject, and the control unit is further configured to perform the steps of selecting a portion of the pulse wave signal indicative of a plurality of heart periods, and, for the portion of the pulse wave signal indicative of a plurality of heart periods, determining a blood pressure variability and/or a blood pressure based on the pulse wave signal of the portion of the pulse wave signal indicative of a plurality of heart periods, determining a respiratory rate variability and/or a respiratory rate based on the pulse wave signal of the portion of the pulse wave signal indicative of a plurality of heart periods, and determining one or more of a heart rhythm, a heart rate variability, and a heart rate based on the pulse wave signal of the portion of the pulse wave signal indicative of a plurality of heart periods.

In a 20th aspect according to the preceding aspect, the portion of the pulse wave signal indicative of a plurality of heart periods is indicative of a plurality of heart periods over a continuous period of at least 1 minute, preferably of at least 3 minutes, more preferably of at least 5 minutes.

In a 21st aspect according to the preceding aspect, the control unit is further configured to perform the steps of determining at least one correlation value based on at least one of the blood pressure variability, the respiratory rate variability, the heart rate variability, and a respective reference value, and determining a medical condition of the subject based on the at least one correlation value.

In a 22nd aspect according to any one of the two preceding aspects, the pulse wave signal indicative of a plurality of heart periods relates to a plurality of heart periods in direct succession to one another.

Advantages of the apparatus for determining photoplethysmographic data representative of vital signals of a subject include that the photoplethysmographic data can be acquired with improved accuracy and/or reliability.

Advantages further include that the apparatus facilitates determining whether a recorded pulse wave fulfills pre-determined quality requirements. Based on this, further processing of the recorded pulse wave can be performed with improved accuracy and/or reliability.

Advantages further include that the apparatus facilitates determining data pertaining to, for example, the heart rhythm, heart rate, respiratory rate, and/or blood pressure of a human subject with improved accuracy and/or reliability.

**Brief description of drawings**

FIG. 1 contains a flow chart of a method for acquiring photopletysmographic data in accordance with the present invention;

FIG. 1A illustrates an exemplary mobile device that can be used in accordance with the method of FIG. 1;

FIG. 1B illustrates an interaction of a human subject with the mobile device shown in FIG. 2;

FIG. 2 illustrates determining signal quality based on determining a perfusion index;

FIGs. 3, 4, and 5 illustrate several steps in determining an SQI based on an acquired pulse wave signal;

FIG. 6 illustrates determining a peak count based on a de-trended pulse wave signal; and

FIGs. 7A and 7B illustrate determining signal quality based on performing a peak count.

**Detailed Description**

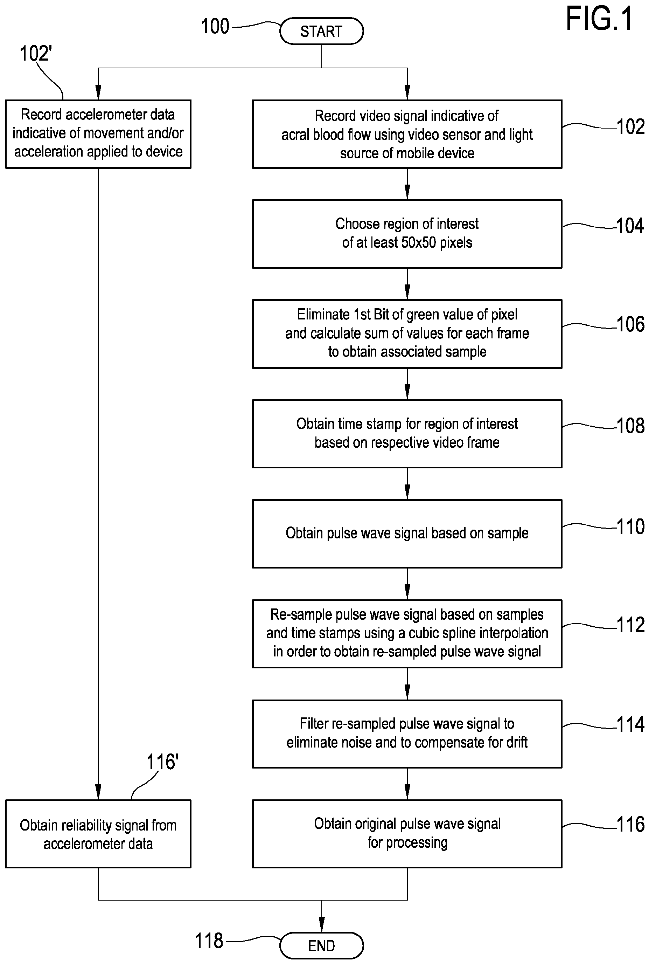

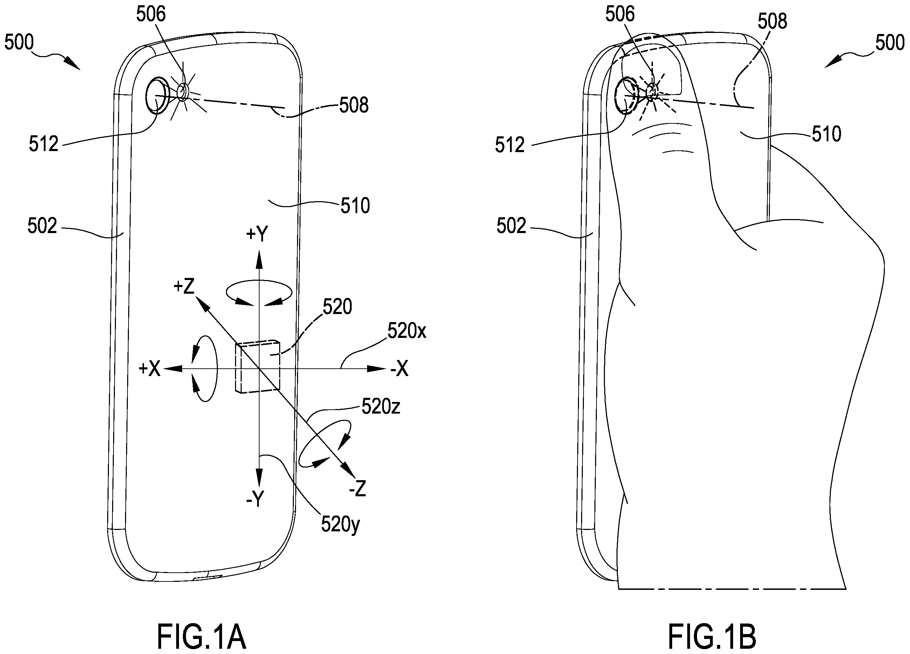

FIG. 1 contains a flow chart of a method 100 for recording pulse wave data in accordance with the present invention, using a mobile device having video recording capabilities. Mobile communication devices, in particular so-called smart phones, have extensive capabilities beyond mere telecommunication. For example, most mobile phones are typically provided with a digital camera capable of capturing still images and video and with a corresponding light source for low-light situations. Generally, a pulse wave can be recorded by detecting, with an optical sensor, light emitted from a light source and reflected by a finger of a subject. In one embodiment, pulse wave data is obtained using a common mobile device equipped with a digital camera (e.g. used as an optical sensor) and an LED light (e.g. used as a light source). The light emitted by the light source is reflected, for example from tissue of a finger placed on the optical sensor and the light source, and the properties of the light (e.g. intensity, hue, brightness, saturation) are affected (e.g. modulated or changed) by acral blood flow in the finger.

Recording pulse wave data in this manner, however, is prone to measurement errors, for example when a subject moves their finger or the device, or when a subject otherwise changes the relative position of the finger and/or the device used. Since the optical measurements are based on very small changes of optical properties of the reflected light (or light permeating through the tissue), already minor changes to measurement parameters may greatly impact the quality of the measurements. For example, the subject could alter a pressure of their finger upon the device, thereby changing an intensity of blood flow through the finger, an intensity or other property of the light reflected by the finger, and/or a degree of ingress of the light into the tissue. Any one of these effects may substantially alter the result of a measurement and, thus, may render recorded pulse wave data useless for further processing. In other instances, changing a position of the extremity (e.g. lifting the arm or changing a position of the hand) may also lead to similar effects and, thus, may render recorded pulse wave data useless for further processing. The same applies to changes to the relative position of the device and the finger.

All the above-mentioned situations typically entail a movement of the device, for example an acceleration (including, e.g., vibration), translation, or rotation so that detecting such a movement can provide information on the reliability of a measurement or a series of measurements. The term "reliability" is understood to be indicative of fitness or suitability for a particular purpose. Within the scope of this document, thus, a reliability signal indicative of a reliability of a signal means that if the reliability signal indicates, for a portion of the signal, that the signal is (sufficiently) reliable, then it can be assumed that the data the (portion of the) signal is pertaining to is (sufficiently) accurate and/or that processing the data the (portion of the) signal is pertaining to will render (sufficiently) accurate results. It is understood that reliability includes any predetermined quality or property indicative of fitness or suitability for a particular purpose. Further, reliability may be quantified as desired, for example by mapping to a series of discrete values (e.g. "good", "bad"), a range of values (e.g. integers from 1 to 10, decimal values such as [0.0,...,1.0], etc.), or any other (numeric) representation allowing for further processing.

Method 100 includes two processes, which are performed substantially simultaneously. The first process (see steps 102' and 116') is performed in order to acquire a reliability signal indicative of a signal quality of the pulse wave signal. The second process (see steps 102 to 116) is performed in order to acquire an original pulse wave signal, which forms the basis for further processing. The reliability signal may include a continuous signal or a series of discrete values over time. In both cases, a reliability value can be determined for any time point, either from the continuous signal or from one or more discrete values (e.g. by interpolation). The two processes are described below.

In the first process, at step 102', data from an accelerometer 520 (see FIG. 1A) is acquired. The accelerometer data may include one or more of acceleration data (e.g. along one or more of an X, a Y, and a Z axis of the mobile device and/or of the accelerometer), movement data (e.g. along one or more of the X, Y, and Z axes), and rotation data (e.g. about one or more of an X, Y, and Z axes).

In one embodiment, accelerometer data is acquired in form of a three-dimensional vector, the vector including acceleration data along the three axes X, Y, and Z. In some embodiments, the accelerometer data may be acquired in form of an n-dimensional vector, the vector including one or more of acceleration, movement, and rotation data.

In the embodiment, in which the accelerometer data is acquired in form of three-dimensional vectors, the acceleration data includes a plurality of three-dimensional vectors. Each vector is provided with a time stamp allowing to relate another time-stamped value or measurement (e.g. a measurement of a parameter made a point in time covered by the plurality of vectors) to a respective vector.

In step 116' a reliability signal is obtained based on the acceleration data. The acceleration data may be processed in a weighted manner, wherein acceleration data pertaining to a particular axis (e.g. X, Y, or Z) may be weighted differently from other axes. In this manner, the acceleration (or movement, or rotation) along one axis may be regarded as more (or less) detrimental to any measurements made and, thus, be weighted with a higher (or lower) factor.

Further, a single magnitude may be obtained for each three-dimensional vector of the plurality of vectors, in order to obtain a single time-stamped value for each vector. This may include calculating a square root based on each vector. This may further include disregarding (single) outliers, which may be inherent to the measured accelerometer data. It is noted that the respective equation, based on which an acceleration value is determined, may depend on, for example, the type of device used. Some devices are provided with standard accelerometers, while some other devices are equipped with more complex integrated sensors, for example integrating an accelerometer, a magnetometer, and/or a gyroscope. Depending on the respective components of a device, a different corresponding method may be employed in order to determine an acceleration value.

In one embodiment, a single acceleration value is determined based on an integrated sensor (including an accelerometer, a magnetometer, and a gyroscope), which provides three acceleration values $x_{Acc}$, $y_{Acc}$, and $z_{Acc}$. The three values are determined at regular intervals, for example once per second, and a value *userMotion* indicative of a motion induced by a user of the device based on the equation: $userMotion = \sqrt{x_{Acc}^2 + y_{Acc}^2 + z_{Acc}^2} *$

$9.81 \frac{m}{s^2}$. This value is provided with a timestamp and stored in a memory unit.

In another embodiment, a single acceleration value is determined based on an accelerometer only, which provides three acceleration values $x_{Acc}$, $y_{Acc}$, and $z_{Acc}$. The three values are determined at regular intervals, for example once per second, and a value *userMotion* indicative of a motion induced by a user of the device based on the equation:

$userMotion = \left\| \sqrt{x_{Acc}^2 + y_{Acc}^2 + z_{Acc}^2} - 9.81 \frac{m}{s^2} \right\|$. This value is provided with a timestamp and stored in a memory unit.

In order to determine the reliability data from the accelerometer data, the accelerometer data, or the modified accelerometer data, are compared to a predetermined threshold value. If the value of the acceleration exceeds the predetermined threshold value, a respective reliability value for the time point corresponding to the time stamp of the accelerometer data exceeding the predetermined value is set to a value associated with the status "unreliable" (e.g. a numerical value, such as "0"). In this manner, the values of the pulse wave data measured or recorded substantially at the same time point can be discarded based on a corresponding reliability value. The reliability data include time-stamped reliability values. A reliability value may include an integer (e.g. 0 = unreliable, 1 = reliable) or a decimal value (e.g. ranging from 0.0 to 1.0, thereby expressing different reliabilities; 0.7 could, thus, be considered relatively reliable). It is noted that any quantitative (e.g. numeric value, values, value range or ranges) or qualitative (e.g. predefined categories, states, or properties) representation may be used in order to define a reliability of the signal.

The above-described embodiment realizes a localized determination of acquired pulse wave data being considered reliable or unreliable. This means, that for each record or data set, a record or data set pertaining to a certain time point, it can be determined whether the record or data set is reliable or unreliable, based solely on a corresponding reliability value associated to the respective time point and without taking into account reliability values determined in adjacent time periods (e.g. before or after the time point). In some embodiments, the reliability values may be averaged over a period of time in order to, for example, filter out outliers or measurement artifacts. In such embodiments, the reliability values may be further be based on interpolated values in order to be able to provide an averaged and/or interpolated reliability value for any point in time - not just respective time points for which accelerometer data has been acquired.

In the second process, at step 102, the subject places their finger on the camera of the mobile device, preferably without touching the light source, such that light emitted from the light source illuminates the acral blood flow and is reflected or dispersed and subsequently detected by the camera. The video signal thus created is recorded and stored in a memory unit of the device. Alternatively, the video signal (e.g. a video stream) can be processed directly, without necessitating storing the pulse wave data in a memory unit. With respect to step 102, it is noted that in some embodiments, an external light source can be used, such that the mobile device need not be provided with a corresponding light source. Such embodiments may be based on an external (artificial or naturally occurring) light source (e.g. an external lamp or sunlight).

In the embodiment described with respect to FIG. 1, the mobile device is a mobile phone provided with a camera and a corresponding light source. In alternative embodiments, the mobile device may be a different device and such different device may be in contact with tissue of a human subject in another manner. For example, the mobile device may include a so-called smart watch provided with an optical sensor and a light source. The light source may be configured to illuminate the tissue of a human subject (e.g. tissue in the region of the wrist of a human subject) and the optical sensor may be configured to detect light reflected from (or permeating through) the tissue in the region of the wrist of the human subject. Irrespective of the individual embodiment, the mobile device may be configured to detect light reflected from (or permeating through) tissue of a human subject, including thumbs or fingers, arms, wrists, ankles, ears, nasal septum, and the forehead of a human subject.

In step 104, a region of interest (ROI) is selected from the full resolution video stream. This selection can be performed, for example, based on brightness information contained in the video stream. In one embodiment, the ROI is determined in a region of maximum brightness within a video frame, off the center and at a minimum distance from the border. This can ensure that a region is chosen that is sufficiently illuminated (e.g. a region close to the light source). In one embodiment, the ROI has a size of at least 50 x 50 pixels (i.e. 2500 square pixels). Generally, the ROI can have a size ranging from 625 to 10000 square pixels, preferably 900 to 6400 square pixels, more preferably 1600 to 3200 square pixels.

In step 106, for the ROI of each frame of the video stream, a sample $s_i$ is calculated, based on

$$s_i = \sum_{j=0}^{N-1} \sum_{k=0}^{M-1} \frac{p(j \cdot w + k)}{2}$$

with p being the value of the green channel of the pixel located within the ROI at the position $j,k$; N and M being the size of the ROI; and w being the width of the ROI. The division by 2 eliminates the lowest Bit of p, such that noise is effectively reduced. This produces a sample $s_i$ for each captured video frame.

In step 108, a time stamp $t_i$ is generated for each sample $s_i$ (more accurately, for each video frame, based on which the sample was calculated) and encoded into the video stream by the video camera. The same time stamp $t_i$ is used in generating the reliability signal. Determining the pulse wave and the measurements based on which the reliability signal is generated, is preferably performed substantially simultaneously.

In step 110, the pulse wave is obtained as a pulse wave signal based on the samples $s_i$ obtained in step 406.

In step 112, a re-sampled pulse wave is obtained by re-sampling the pulse wave from the samples $s_i$ (i.e. as obtained in step 410) based on the associated time stamps obtained in step 408. This is necessary due to technical issues in detecting, generating, and encoding video data, for example resulting in dropped frames or non-constant frame rates. Based on these issues, the samples $s_i$ cannot be obtained at fixed and reliable time intervals. In order to obtain the re-sampled pulse wave, the pulse wave is re-sampled using a cubic spline interpolation and is performed on each polynomial. Here, two subsequent samples are interpolated by a third-degree polynomial. The position (in time) of the samples corresponds to the time stamps. The polynomial $S_i$ for the range $[t_i, t_{i+1}]$ is calculated as follows:

$$S_i = a_i + b_i(t - t_i)^2 + d_i(t - t_i)^3$$

with $i = 1, ..., n\text{-}1$. The process of re-sampling includes incrementing t continuously by 1 ms, corresponding to a sample rate of 1000 Hz. The parameters $a_i$, $b_i$, $c_i$, and $d_i$ have to be set to suitable values. The pulse wave is obtained as the signal S being the result of the re-sampling.

In step 114, the re-sampled pulse wave is filtered to eliminate noise and to compensate for drift. This can be achieved by applying a common bandpass filter (e.g. 0.1 to 10 Hz).

In step 116, the original pulse wave signal is obtained as a basis for further processing. When the second process ends, for example when a desired pulse wave signal has been obtained, also the first process ends, so that the reliability signal obtained in the first process pertains to substantially the same time period as the pulse wave signal obtained in the second process.

FIG. 1A illustrates an exemplary mobile device that can be used in accordance with the method of FIG. 1. The mobile device 500 has a frame or main body 502 and a device panel 510. In some examples, the device panel 510 can be a back panel of the mobile device 500. The device 500 further has a camera device 512 capable of detecting digital video signals, for example in the form of digital still images and digital video. The camera device 512 is configured to detect video signals representative of objects located generally with a frustum-shaped region along a main detection direction 508. The device 500 further has a light source 506 configured to illuminate any objects located in front of camera device 512, i.e. located within the frustum-shaped region and/or along a main detection direction 508.

Mobile device 500 further comprises a light source 506. The light source 506 can be configured to provide both a single flash of light and a continuous light beam, depending on a mode of operation. When recording video, the light source typically provides a continuous light beam. An object placed within the view of camera device 512 will reflect and/or diffuse light emitted from light source 506, so that the reflected light can be detected by camera device 512.

Mobile device 500 further comprises a control unit (e.g. a CPU, micro processor, SoC; not shown) coupled to other components, such as camera device 512, light source 506, a memory unit, a user interface, input means, an audio unit, a video unit, a display, and other.

Mobile device 500 further comprises a sensor, typically an accelerometer or other type of sensor configured for detecting a physical parameter, for example including acceleration, motion, rotation, and orientation. Typically, the sensor includes an accelerometer 520. However, the sensor may further or alternatively include any sensor configured to detect acceleration, motion, orientation and/or rotation. Thus, the motion sensor may include an accelerometer 520, a magnetometer, a gyroscope (or gyro), or other sensor configured to detect acceleration, motion, orientation and/or rotation. Suitable sensors are typically provided in the form of micro-electromechanical systems or MEMS.

A magnetometer may be configured to detect a magnetic field, for example the magnetic field of the earth. The signal generated by a magnetometer may be used in order to detect acceleration, motion, orientation and/or rotation of an associated device.

An accelerometer may be configured to detect proper acceleration (as opposed to coordinate acceleration),

**EP 3 403 574 A1**

i.e. absolute acceleration, such that an accelerometer resting on a fixed surface may detect acceleration due to the earth's gravity in direction of one or more of it's axes, depending upon an orientation of the accelerometer. Thus, an orientation of the accelerometer may be determined based on the acceleration detected due to changes in the direction of the earth's gravity. The signal generated by an accelerometer may, thus, be used in order to detect acceleration, motion, orientation, and/or rotation of an associated device.

A gyroscope may be configured to detect movement in terms of acceleration and/or rotation. The signal generated by a gyroscope may be used in order to detect acceleration, motion, orientation and/or rotation of an associated device.

In the embodiments described, the mobile device 500 is provided with an accelerometer 520. However, embodiments of the present invention may be based on the mobile device being provided with an accelerometer 520 as described and/or on one or more sensors (e.g. including one or more of a magnetometer, a gyroscope, an accelerometer, or other sensor as described above) configured for detecting acceleration, motion, orientation and/or rotation of the mobile device 500, thus, not limiting the inventive concepts on the mobile device being provided with an accelerometer 520. Accelerometers are typically available as single- or multi-axis accelerometers and enable the detection of magnitude and direction of proper acceleration, as a vector quantity.

In the embodiment shown in FIG. 1A, mobile device 500 is provided with an accelerometer 520 configured to detect acceleration along three axes, namely an X-axis 520x, a Y-axis 520y, and a Z-axis 520z. The directions of the axes 520x, 520y, and 520z, as well as a polarity thereof (i.e. defining which direction, e.g., denotes +X and which -X) may differ from what is shown in FIG. 1A, and the type, position, and orientation of accelerometer 520 within mobile device 500 may vary. In other embodiments, other accelerometers may be used, for example accelerometers having more or less axes.

Typically, mobile devices are provided with a single accelerometer 520. In some embodiments, mobile devices may be provided with two or more accelerometers. For example, it might be desired to provide mobile device 500 with a first accelerometer that provides a first accuracy, detection range, resolution, and/or number of detection axes, and with a second accelerometer that provides a second accuracy, detection range, resolution, and/or number of detection axes different from the first. In such examples, the first (or second) accelerometer may offer a lower power consumption as compared to the other, so that one of the first and second accelerometers may be selected based on a use configuration of the mobile device. It is noted that embodiments of the present invention may be based on any accelerometer that is configured to detect acceleration, irrespective of accuracy, detection range, resolution, and/or number of detection axes. Preferred embodiments, however, are based on accelerometers having at least three axes of detection.

FIG. 1B illustrates an interaction of a human subject with the mobile device shown in FIG. 1A. In order to take a measurement, the subject places a finger (e.g. a thumb) on mobile device 500, covering both the camera device 512 and the light source 506. The individual configuration of the mobile device (e.g. a position of camera device 512 and light source 506 or the distance in between) is of secondary relevance, as long as it is physically possible to cover both the camera device 512 and the light source 506 with a suitable extremity (e.g. finger, thumb, ear). In this respect, any extremity suitable for (acral) measurement can be used in accordance with the present invention. In general, any body part that is associated with pulsating blood flow can be used in accordance with the present invention, as long as a meaningful signal indicative of the blood flow can be detected via the body part of a subject (e.g. fingers, arms, wrists, ankles, ears, nasal septum, or forehead).

In some embodiments, the control unit of mobile device 500 will process signals provided by camera device 512 and detect, based on the signals provided, that one or more parameters indicative of video quality (e.g. brightness, contrast, focus) are outside of preferred operating ranges due to a low-light and/or close-proximity situation created by the placement of the thumb directly onto camera device 512. The control unit may then provide control signals to one or more components, for example to light source 506, in order to make adjustments to the parameters (e.g. activating light source 506 in order to compensate for low light).

Upon placement of the suitable extremity (here, e.g., the thumb of the subject), the measurement is initiated by activating the light source 506 to emit a continuous light beam of sufficient intensity, such that acral blood flow is illuminated. At substantially the same time, camera device 512 is activated and the light reflected by the acral blood flow is detected by camera device 512. Both activating the light source 506 and activating the camera device 512 can be achieved by corresponding program code executed by the control unit comprised in device 500.

The activation can be triggered manually, for example by selecting a corresponding function on a user interface of device 500, or automatically, for example triggered by a sensor (e.g. a proximity sensor, an optical sensor), a timer, voice recognition, or other (input means). In one example, the signal of the sensor is continuously processed to check for the presence of a suitable signal. Video data is then recorded or transmitted for further processing for a predetermined period of time, typically ranging from several seconds to 5 minutes. In preferred embodiments, the predetermined period of time ranges from 1 minute to 5 minutes, more preferably the predetermined period of time is about 1 minute, about 3 minutes, or about 5 minutes. Selecting such predetermined time periods allows for a reliable determination of a subject's heart rhythm.

In some alternative embodiments, the time period is not predetermined, but determined as the recording/transmitting is ongoing, in that a quality measure is calculated from the recorded/transmitted data and the recording/transmitting is performed until a sufficient number of heart periods (e.g. 60-400) of sufficient quality (see further details below) has been recorded/transmitted, in order to determine a subject's heart rhythm. Completion of the recording/transmitting can be indicated to the subject, for example, by an acoustic and/or optical signal emitted by an audio and/or video component of device 500.

At substantially the same time, the control unit is configured to acquire accelerometer data from the accelerometer 520, the accelerometer data being indicative of one or more of an acceleration, an orientation, a motion, and a rotation of accelerometer 520, and, thus, of mobile device 500.

It is noted that other embodiments employ the same or different sensors and/or devices. For example, smart watches having a corresponding light source/sensor assembly as described above with respect to FIGs. 1A and 1B, can be used as well. These devices have an advantage in that the sensor is kept in close proximity to the body (here, e.g. the wrist) of a subject, thereby facilitating continuous measurements and/or measurements of arbitrary duration and at arbitrary time points, without interaction of a subject (e.g. also during sleep). It is noted that the above concepts apply to a range of sensors and are not limited to a particular or otherwise specific embodiment of sensor hardware.

As described above, acquiring the pulse wave signal and the reliability signal allows for determining whether the pulse wave signal is reliable, based on the reliability signal. Therefore, the combination of the pulse wave signal and the reliability signal is already inherently reliable. Also, the control unit 530 may be configured to determine a verified pulse wave signal based on the pulse wave signal and the reliability signal, for example including discarding the pulse wave signal for a respective time period, if the reliability signal indicative of the reliability of the pulse wave signal for the respective time period is not within a predetermined range (or below a predetermined threshold value). The pulse wave signal being discarded would, thus, indicate that the pulse wave signal is not regarded as reliable and/or indicate that there is a high probability of the pulse wave signal containing artifacts or otherwise being inaccurate.

However, other, alternative, or additional methods can be applied in combination with the above-described method for obtaining a reliable signal in order to further improve the reliability and/or quality of the signal, and/or in order to avoid recording of a compromised signal. In the following, three further methods are described, which can be combined with the above-described method for obtaining a reliable signal in any combination of the three methods.

The first method is based on determining a correlation of subsequent heart periods and on determining signal quality based on whether the determined correlation is below a minimum correlation value. To this aim, an original pulse wave signal is typically pre-processed. Pre-processing may involve one or more of determining a trend and subsequently de-trending the original pulse wave (see below with respect to the second method), applying a low-pass filter, and determining heart periods.

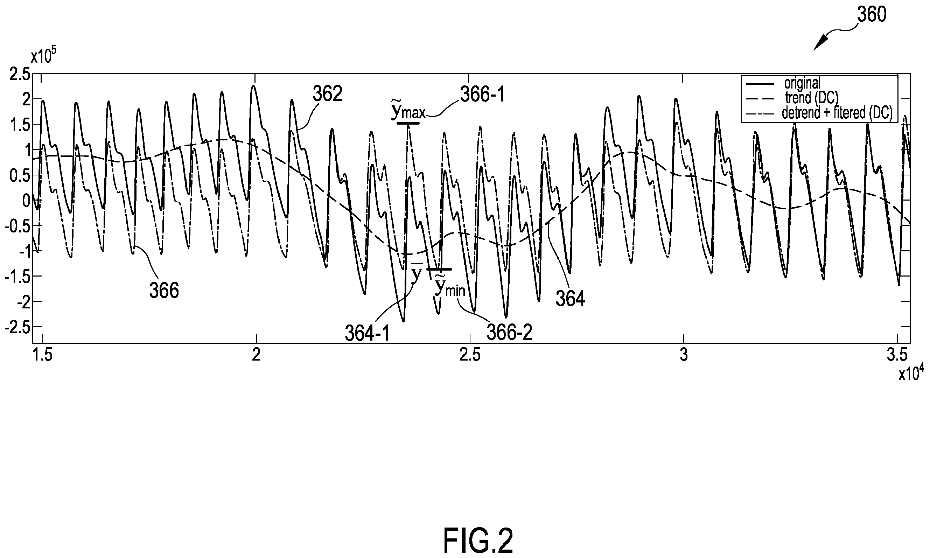

Determining a trend and de-trending the original pulse wave may include observing a running window of three subsequent heart periods calculating non-pulsatile blood as the statistical mean over the running window. This is described in more detail below with respect to FIG. 2. As a result, a trend is obtained, which represents low frequency variations of the original pulse wave. Additionally or alternatively, a low-pass filter may be applied in order to filter out unwanted frequencies that are outside of frequencies of interest (e.g. outside of a range of 0.5 Hz to 7 Hz). A low-pass filter can substantially attenuate or eliminate frequencies above a predetermined frequency, for example above 3 Hz. Heart periods are typically determined by determining inflection points in the original pulse wave.

Optionally, in order to determine the correlation of subsequent heart periods, each of a selected number of periods may be normalized into an interval of y-values between 0 and 1. In one embodiment, a window of three subsequent heart periods is observed. Generally, using larger windows may increase specificity while using smaller windows may increase sensitivity. The correlation of the three subsequent heart periods is based on a pairwise comparison of the (normalized) periods (e.g. 1-2, 1-3, 2-3) and on determining a similarity score for each pair (localized similarity). Next the maximum of the resulting correlation coefficients is determined and compared to a threshold value. If the maximum correlation coefficient is below the threshold value, the original pulse wave, for the respective heart periods (or for the respective three heart periods) can be discarded or disregarded as being of insufficient quality.

The second method is based on determining a perfusion index using a running window over subsequent heart periods. The perfusion index is the ratio of the pulsatile (AC) blood flow to the non-pulsatile (DC) or static blood in peripheral tissue. In other words, it is the difference of the amount of light absorbed through the pulse when light is transmitted through the finger. Signal quality is determined based on a ratio of AC and DC, a smaller ratio being indicative of a signal of lesser quality.

FIG. 2 illustrates determining signal quality based on determining a perfusion index. The diagram 360 depicted in FIG. 2 shows several graphs 362, 364, and 366 illustrating the second method. Diagram 360 illustrates the amplitude of signal (see vertical axis) over time (see horizontal axis). Graph 362 is indicative of the original pulse wave signal. In one embodiment, a running window of three or more subsequent heart periods is observed and DC is calculated as the statistical mean over the running window. In other embodiments, windows of a different size can be observed, in particular windows having a size of three or more heart periods. Subsequent values of DC determine a trend of the signal, shown in diagram 360 as graph 364. As can be seen, the trend is interpolated in order to provide a continuous signal. Based on the original pulse wave 362 and the trend 364, a de-trended and filtered pulse wave signal 366 is determined. In order to determine the quality index, $\frac{AC}{DC}$ is calculated as $\frac{\tilde{y}_{max} - \tilde{y}_{min}}{\bar{\tilde{y}}}$, with $\tilde{y}_{max}$ and $\tilde{y}_{min}$ respectively corresponding to the maximum and minimum values of signal 366 within a single heart period, and y corresponds to the value of the trend 364 for the heart period, as previously determined for the pulse wave signal 362. In order to determine whether the pulse wave signal for a particular heart period fulfills quality requirements, the ratio $\frac{AC}{DC}$ is compared to a threshold value. If the ratio is below the threshold value, the pulse wave signal for the heart period in question is not of sufficient quality.

The third method is based on determining respective maxima in the frequency spectrum of a pulse wave signal, when observing a running window of three subsequent heart periods. The method is based on a Fourier Transformation (e.g. an FFT based on a Hanning window) and includes determining respective maxima in the resulting frequency spectrum. The frequency spectrum of a pulse wave signal of good quality will exhibit few peaks and significant (i.e. well visible) harmonic waves without secondary peaks. If the frequency spectrum suffers from a large number of peaks, this indicates that the original pulse wave is of low quality due to several frequencies dominating the spectrum.

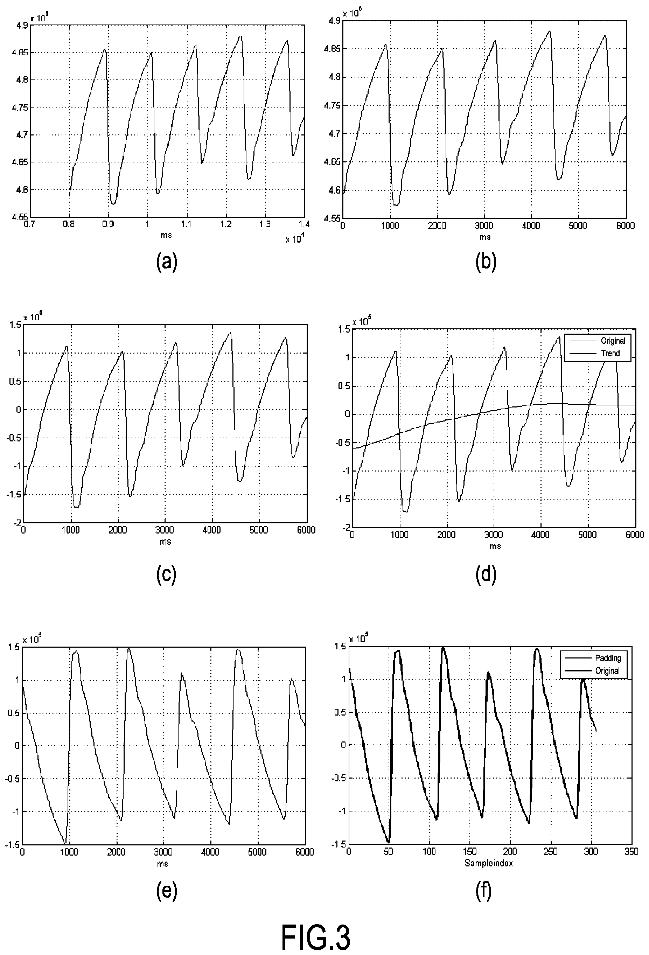

FIGs. 3, 4, and 5 illustrate several steps in determining an SQI based on an acquired pulse wave signal. In general, determining the SQI is based on maintaining a buffer of size w, which stores samples of an acquired pulse wave along with corresponding time stamps. The buffer is typically implemented as a ring buffer, corresponding to a moving window of the size w. The example shown in FIGs. 3, 4, and 5 is based on a window size $w = 6 \cdot FS$, wherein FS is the sampling rate. The horizontal axis in each diagram shows time in milliseconds (ms) and the vertical axis shows the sample value.

FIG. 3 (a) shows an unprocessed raw signal, which is generated based on the sample values and the associated time stamps. Then, as shown in FIG. 3 (b), the initial time stamp is set to zero, so that the signal is reset in time. Further, the signal is re-sampled based on cubic splines using a sampling rate of 50 Hz in order to ensure that the signal has exhibits equidistant sampling. In detail, the cubic spline approximation results in each two subsequent samples being approximated using a 3rd order polynomial. The relative position (in time) of the samples corresponds to the respective time stamps. The polynomial $S_i$ in the range $[t_i, t_{i+1}]$ is determined based on $S_i = a_i + b_i(t - t_i) + c_i(t - t_i)^2 + d_i(t - t_i)^3$, with $i = 1, ..., N - 1$. In accordance with the re-sampling rate of 50 Hz, t is continuously incremented by 20 ms. Parameters $a_i$, $b_i$, $c_i$, $d_i$ must be determined in a suitable manner. The signal shown in FIG. 3 (b) forms the basis for the following processing steps.

FIG. 3 (c) illustrates the signal after normalization (e.g. zero-mean, optionally with unit variance; see different range of values on the vertical axis in FIG. 3 (b) as compared to FIG. 3 (c)). Subsequently, the signal is de-trended, see FIG. 3 (d). To this aim, a Gaussian low-pass filter is applied in order to filter the signal and to find a trend, based on $S_i = S_i - \bar{S}$, with $i = 0, ..., n - 1$, and based on $\bar{S} = \frac{1}{n} \sum_{i=0}^{n-1} S_i$. FIG. 3 (d) illustrates the original signal and the trend determined from the signal. As illustrated in FIG. 3 (e), the trend is subtracted from the original signal based on $S_i = -(S_i - tr_i)$, with $i = 0, ..., n - 1$, $tr_i$ corresponding to a respective sample point in the trend signal. Further, the signal is inverted (see FIG. 3 (e)).

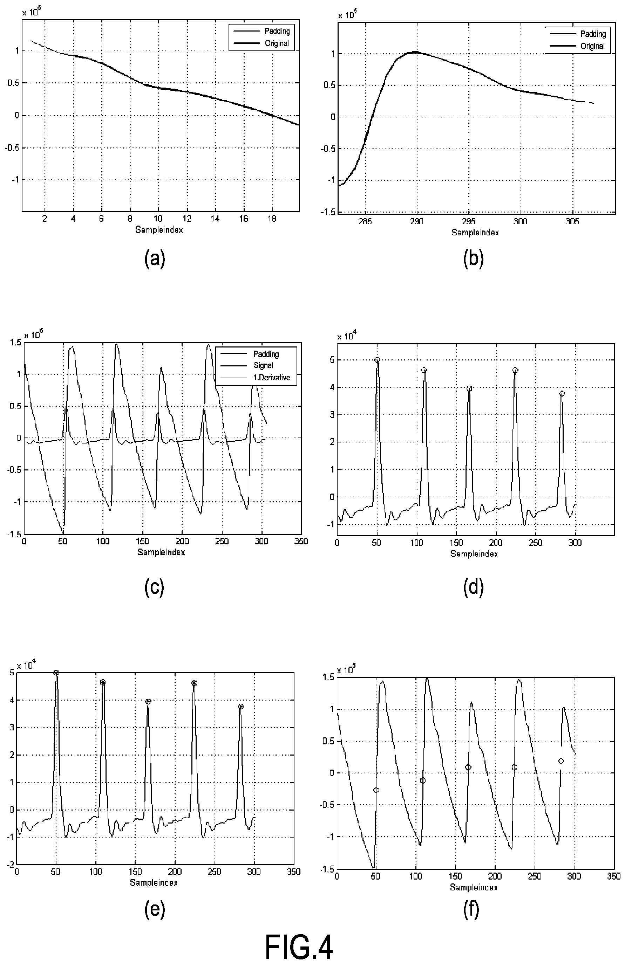

In order to find periods, the positive maxima of the 1st order derivative of the de-trended signal are determined. These positive maxima correspond to the inflection points in the rising edges. Determining the 1st order derivative may be achieved based on the algorithm of Savitzky-Golay, which causes a simultaneous smoothing of the derivative, as illustrated in FIG. 3 (f). The boundaries may be expanded by padding (see FIG. 4(a) for the left boundary and FIG. 4(b) for the right boundary). It is noted that the horizontal axis in FIGs. 3 (f), 4 (a), et seq. shows the sample index and, thus, effectively sets the unit size to 20 ms (based on the sample rate of 50 Hz). Smoothing and the 1. order derivative are calculated using the Savitzky-Golay filter: $Y_j = \sum_{i=-\frac{m-1}{2}}^{\frac{m-1}{2}} C_i S_{j-i}$, with $\frac{m+1}{s} \leq j \leq n - \frac{m-1}{2}$. Further, $j = 1, ..., n$, wherein $n$ is the number of sampled points. $C_i$ denotes the convolution kernel and, here, C = (-3, -2, -1, 0, 1, 2, 3). Further, m is the number of coefficients of the convolution kernel (here, $m=7$). In order to provide point at either end, the signal is continued, wherein $\frac{m+1}{2}$ points have to be expanded at both ends (see FIG. 4 (a) and (b)): $S_{-i} = 2S_0 - S_i$ and $S_{n-1+i} = 2S_{n-1} - S_{n-1-i}$, with $i = 1, ..., 4$. Negative indices of S and indices larger than $n - 1$ are indicative of the expansion of the signal. FIG. 4 (c) illustrates the original signal, the padded signal, and the 1st order derivative of an illustrative example.

In order to determine the periods, all positive maxima of the 1st order derivative have to be identified (see FIG. 4 (d)). Subsequently, the identified maxima are sorted by value, largest first, in descending order. Then 25% of both the largest and smallest values are discarded and mean value of the remaining 50% is determined. One third of the mean value is then taken as a threshold value for maxima of the 1st order derivative and all maxima exceeding this threshold value are taken into account when marking the beginning of the periods (see FIG. 4 (e)). Each period is enclosed between two subsequent maxima of the 1st order derivative (see FIG. 4 (f)).

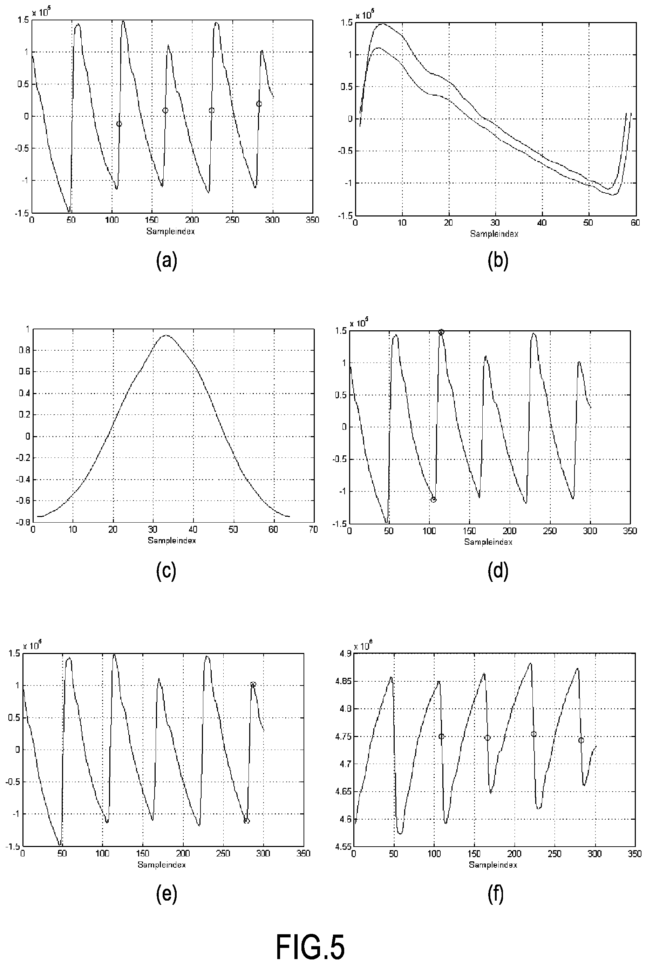

From the periods determined as described above, the last three periods are selected for a comparison step (see FIG. 5 (a)). In this comparison step, each of the three selected periods is compared with the two other periods (i.e. 1-2, 1-3, 2-3; see FIG. 5 (b)) and a correlation (or similarity) value is determined using a cross correlation function (see FIG. 5 (c)), wherein temporal lag is disregarded. Out of the three determined correlation values, the maximum value is taken as being indicative of the similarity for the respective window. The normalized cross correlation can be determined as follows. With $x$ and $y$ being the periods to compare, the length of the periods is assumed as being 64 units (longer

$$k_{xy} = \max\left\{\frac{F^{-1}\{F\{x\} * F\{y\}^*\}}{\|x\|_2 \|y\|_2}\right\},$$

periods are cut and shorter periods are padded accordingly): with F being the FFT of fixed length, * being the conjugate. Consequently, the similarity value of the window is $SQI_{corr} = max\{k_{12}, k_{13}, k_{23}\}$.

The beginnings of the periods determined in the comparison step, i.e. the inflection points as illustrated in FIG. 5 (a), form the basis for determining the perfusion index. Each of these points marks the rising flank of the periods and for each of these points, the previous maximum (i.e. to the left of the respective point) and the following maximum (i.e. to the right of the point) are determined, as shown in FIG. 5 (d) and (e). The inflection point is then projected into the original pulse wave (before de-trending), which provides the estimate for the DC (i.e. the non-pulsatile blood flow), as illustrated in FIG. 5 (f). In order not to let the DC range get too small, a minimum value of 1000 is set. The following

$$perfIdx_i = \frac{AC_i}{DC_i}.$$

equation determines the perfusion index for a period $i$: Subsequently, the mean value $\overline{perfIdx}$ of the mid 50% of the ordered values (see above; upper and lower 25% are disregarded) is determined as the overall $perfIdx$. $SQI_{perfindex} = 100 \cdot \overline{perfIdx}$.

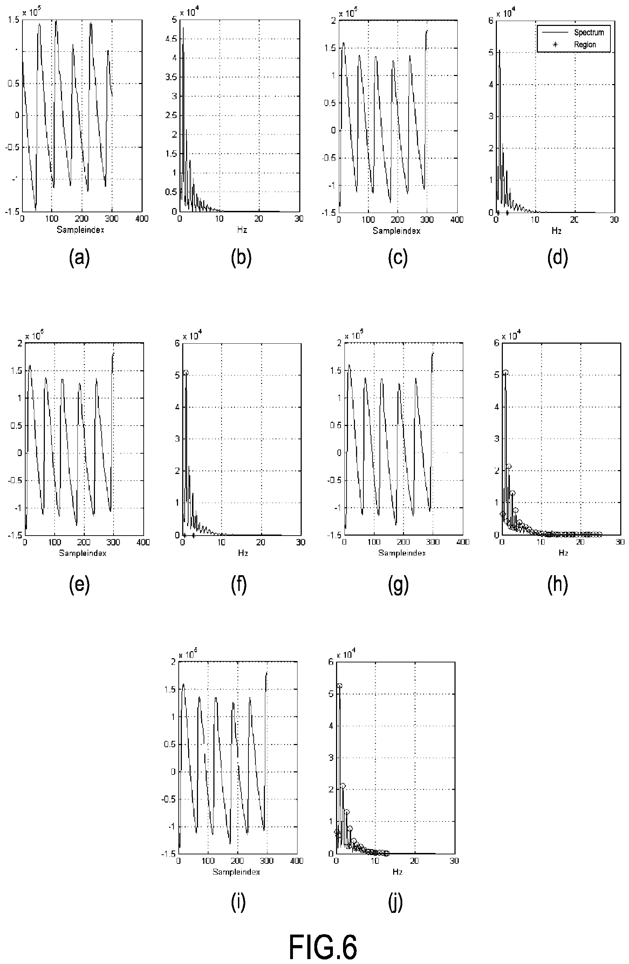

FIG. 6 illustrates determining a peak count based on the de-trended pulse wave signal. The de-trended signal forms the basis for determining the number of dominant frequencies. The frequency spectrum is generated based on

$$sp = \left|\frac{F\{S \cdot hanning(N)\}}{NFFT}\right|.$$

the following equation, with N being the length of the signal and $NFFT$ the next power of two: Here, S is multiplied with the Hanning window of length N. Subsequently, the vector is set to length $NFFT$ by zero padding. Over this vector, the FFT is generated and normalized (see FIG. 6 (a) and (b); diagram (a) shows the de-trended signal and diagram (b) shows the corresponding frequency spectrum).

In order to determine the heart rate from the frequency spectrum, a frequency region is defined, in which the highest peak is determined (see FIG. 6 (c) and (d)). The frequency of the highest peak corresponds to the current heart rate (see FIG. 6 (e) and (f)). The frequency region (or search region) is defined as 0.6 Hz to 3.0 Hz.

In order to determine the number of dominant frequencies, the spectrum sp is examined for all occurring peaks

$idx$ (see FIG. 6 (g) and (h)), with $idx$ corresponding to the indices of the peaks in the spectrum: $sp(idx) = \frac{sp(idx)}{\sum_{idx} sp(idx)}$. Each peak is divided by the sum of all peaks. The threshold value based on which the remaining peaks are separated is determined as: $thr = \frac{sp(idxMax)}{500}$, with $idxMax$ corresponding to the index of the highest peak. All peaks larger than the threshold value thr are taken into account when determining the number of the dominant frequencies (see FIG. 6 (i) and (j)): $SQI_{peakcount} = \#(sp(idx) > thr)$. The overall SQI is then determined from the discriminant analysis of the three parameters. An example SQI determined from an example data set was determined as:

$$SQI = 3.47723899802885 \cdot SQI_{corr} - 0.125428815152482 \cdot SQI_{peakcount} +$$
$$0.408789084581875 \cdot SQI_{perfindex} + 0.920673015347965.$$

Typically, the values determined based on the above discriminant function has to be mapped onto the interval (0,1). Based on this, an indicator can be determined as a visual tool for estimating signal quality. Possible candidate include, but re not limited to, linear mappings or n-th-order polynomials focusing on individual regions separately.

It is noted that depending on respective implementations of the process (e.g. depending on the devices and/or operating systems used), individual discriminant functions and/or mappings may have to be provided. Further, the parameter $SQI_{peakcount}$ does not take into account the current heart frequency.

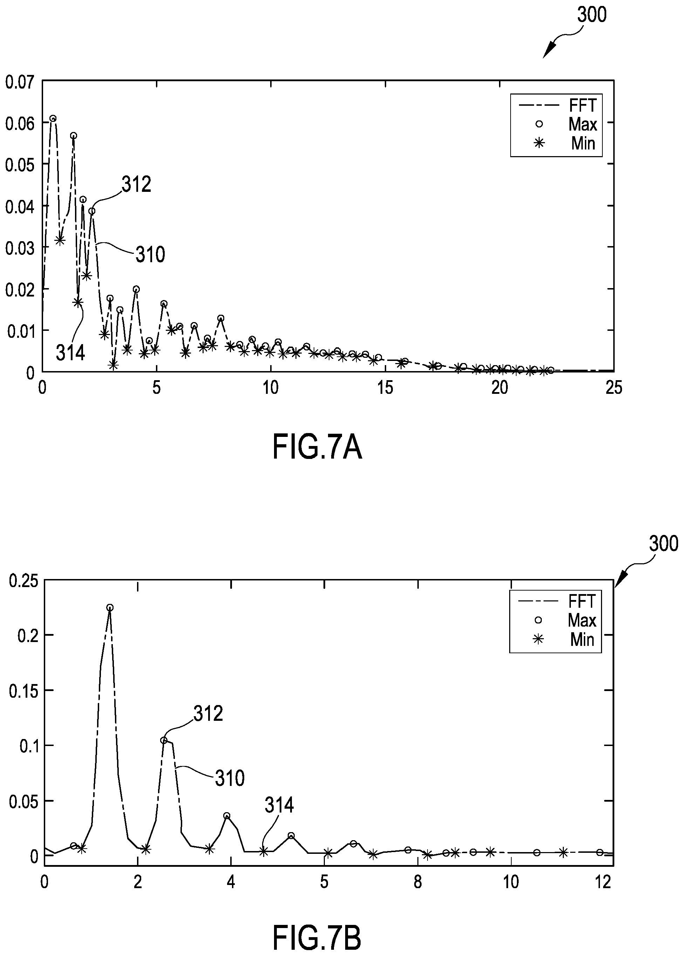

FIGs. 7A and 7B illustrate determining signal quality based on performing a peak count. As described above, a Fourier Transformation is performed and provides a frequency spectrum, for example, as shown in FIGs. 7A and 7B. Each diagram shows the frequency spectrum as FFT (see vertical axis) over frequency (see horizontal axis; Hz). FIG. 7A illustrates a resulting frequency spectrum indicative of an original pulse wave of low quality. Diagram 300 in FIG. 7A shows the resulting graph 310 and respective minima 314 and maxima 312. As can be seen from FIG. 7A, the graph 310 exhibits a number of peaks, for example, about eight peaks in the frequency band below 5 Hz and a similar number for the frequency band from 5 Hz to 10 Hz. A comparatively high number of peaks indicates that the original pulse wave for the three heart periods observed was of low quality.

As a comparative example, FIG. 7B illustrates a resulting frequency spectrum indicative of an original pulse wave of high quality. Diagram 300 in FIG. 7B, again, shows the resulting graph 310 and respective minima 314 and maxima 312. As can be seen from FIG. 7B, the graph 310 exhibits a smaller number of peaks as compared to FIG. 7A. In the comparative example of FIG. 7B, graph 310 exhibits only about four peaks in the frequency band below 5 Hz and a similar number for the frequency band from 5 Hz to 10 Hz. A comparatively low number of peaks indicates that the original pulse wave for the three heart periods observed was of high quality.

While the invention has been described in connection with what is presently considered to be the most practical and preferred embodiments, it is to be understood that the invention is not to be limited to the disclosed embodiments, but on the contrary, is intended to cover various modifications and equivalent arrangements included within the spirit and the scope of the appended claims.

**Claims**

1. An apparatus (500) for determining a pulse wave signal representative of vital signs of a subject, the apparatus comprising:

   a control unit (530);
   a first sensor (512) coupled to the control unit and configured for emitting a first signal indicative of a pulse wave of a subject;
   a second sensor (520) coupled to the control unit and configured for detecting motion the apparatus is subjected to and for emitting a second signal based on the detected motion; wherein
   the control unit is configured to:

      receive the first signal from the first sensor (512);
      determine a pulse wave signal based on the first signal;
      receive the second signal from the second sensor (520); and
      determine a reliability signal based on the second signal, the reliability signal being indicative of a reliability of the first signal.

2. The apparatus of the preceding claim, wherein the reliability signal is further indicative of a reliability of the pulse wave signal; and optionally wherein:

   - the control unit (530) is further configured to determine one or more correlation values based on the pulse wave signal; and
   - determining the reliability signal is further based on the one or more correlation values.

3. The apparatus of the preceding claim, wherein:

   the control unit (530) is further configured to determine one or more perfusion indices based on the pulse wave signal; and
   determining the reliability signal is further based on the one or more perfusion indices.

4. The apparatus of any one of the two preceding claims, wherein:

   the control unit (530) is further configured to determine one or more frequency spectra based on the pulse wave signal; and
   determining the reliability signal is further based on the one or more frequency spectra.

**5.** The apparatus of any one of the preceding claims, wherein the control unit (530) is further configured to determine a verified pulse wave signal based on the pulse wave signal and the reliability signal; optionally wherein determining the verified pulse wave signal comprises one or more of:

- selectively discarding one or more portions of the pulse wave signal based on the reliability signal and determining the verified pulse wave signal based on the pulse wave signal without the discarded one or more portions of the pulse wave signal; and
- selectively selecting one or more portions of the pulse wave signal based on the reliability signal and determining the verified pulse wave signal based on the selected one or more portions of the pulse wave signal.

**6.** The apparatus of the preceding claim, wherein determining the verified pulse wave signal comprises:

selectively assigning a signal quality index (SQI) to one or more portions of the pulse wave signal based on the reliability signal; wherein

determining the verified pulse wave signal is exclusively based on the one or more portions of the pulse wave signal where each of the one or more portions of the pulse wave signal has assigned thereto a signal quality index fulfilling a minimum requirement; optionally wherein

- the signal quality index comprising one or more of:

- one or more discrete values, the one or more discrete values optionally being selected from a predetermined set of discrete values; and
- a numeric value, the numeric value optionally falling within a predetermined numeric range ranging from a minimum value to a maximum value; and/or

- fulfilling a minimum requirement includes one or more of:

- exceeding a minimum value,
- falling within a range defined by a minimum value and a maximum value, and
- not exceeding a maximum value.

**7.** The apparatus of any one of the preceding claims, wherein the second sensor (520) is configured to detect one or more of:

a first acceleration along a first axis (520x, 520y, 520z);
a second acceleration along a second axis (520x, 520y, 520z);
a third acceleration along a third axis (520x, 520y, 520z);
a first rotation about the first axis (520x, 520y, 520z);
a second rotation about the second axis (520x, 520y, 520z); and
a third rotation about the third axis (520x, 520y, 520z).

**8.** The apparatus of the preceding claim, wherein:

determining the reliability signal is further based on the first, second, and/or third acceleration; optionally wherein determining the reliability signal includes determining whether the first, second, and/or third acceleration exceeds a predetermined acceleration threshold value; and/or
determining the reliability signal is further based on the first, second, and/or third rotation; optionally wherein determining the reliability signal includes determining whether the first, second, and/or third rotation exceeds a predetermined rotation threshold value.

**9.** The apparatus of any one of the preceding claims, wherein:

the apparatus further comprises a main body (502) configured to carry the control unit, the first sensor (512), and the second sensor (520); and/or
the first sensor (512) is configured for detecting light reflected from and/or permeating through tissue of the subject; and wherein emitting the first signal is based on the detected light; and/or
the first sensor includes one or more of an optical sensor, a CCD sensor, a heart rate monitor (HRM); and/or
the second sensor includes one or more of an accelerometer, a magnetometer, and a gyroscope.

10. The apparatus of the preceding claim, further comprising a light source (506) coupled to the control unit (530) and configured to illuminate tissue of the subject; optionally wherein the control unit is configured to control the light source to selectively illuminate tissue of the subject; optionally wherein:

- the light source (506) is arranged in close proximity to the first sensor (512); and/or
- the light source (506) is configured for illuminating tissue of the subject positioned in close proximity or in contact with the first sensor (512).

11. The apparatus of any one of the preceding claims, wherein the control unit (530) is configured to:

control the first sensor (512) to emit the first signal; and/or
control the second sensor (520) to emit the second signal.

12. The apparatus (500) of any one of the preceding claims, wherein:

the pulse wave signal is representative of a heart beat of the subject; and
the control unit (530) is further configured to perform the steps of:

selecting a portion of the pulse wave signal indicative of a plurality of heart periods;
for the portion of the pulse wave signal indicative of a plurality of heart periods:

- determining a blood pressure variability and/or a blood pressure based on the pulse wave signal of the portion of the pulse wave signal indicative of a plurality of heart periods;
- determining a respiratory rate variability and/or a respiratory rate based on the pulse wave signal of the portion of the pulse wave signal indicative of a plurality of heart periods; and
- determining one or more of a heart rhythm, a heart rate variability, and a heart rate based on the pulse wave signal of the portion of the pulse wave signal indicative of a plurality of heart periods.

13. The apparatus (500) of the preceding claim, wherein the portion of the pulse wave signal indicative of a plurality of heart periods is indicative of a plurality of heart periods over a continuous period of at least 1 minute, preferably of at least 3 minutes, more preferably of at least 5 minutes.

14. The apparatus (500) of any one of the preceding claims, wherein the control unit (530) is further configured to perform the steps of:

determining at least one correlation value based on at least one of the blood pressure variability, the respiratory rate variability, the heart rate variability, and a respective reference value; and
determining a medical condition of the subject based on the at least one correlation value.

15. The apparatus of any one of the two preceding claims, wherein the pulse wave signal indicative of a plurality of heart periods relates to a plurality of heart periods in direct succession to one another.

# FIG.1

```
100 —( START )

102'
┌─────────────────────────┐        ┌─────────────────────────────┐
│ Record accelerometer data│        │ Record video signal indicative of│——102
│ indicative of movement and/or│    │ acral blood flow using video sensor and light│
│ acceleration applied to device│   │ source of mobile device │
└─────────────────────────┘        └─────────────────────────────┘
                                                 │
                                    ┌─────────────────────────────┐
                                    │ Choose region of interest    │——104
                                    │ of at least 50x50 pixels     │
                                    └─────────────────────────────┘
                                                 │
                                    ┌─────────────────────────────┐
                                    │ Eliminate 1st Bit of green value of pixel│——106
                                    │ and calculate sum of values for each frame│
                                    │ to obtain associated sample  │
                                    └─────────────────────────────┘
                                                 │
                                    ┌─────────────────────────────┐
                                    │ Obtain time stamp for region of interest│——108
                                    │ based on respective video frame│
                                    └─────────────────────────────┘
                                                 │
                                    ┌─────────────────────────────┐
                                    │ Obtain pulse wave signal based on sample│——110
                                    └─────────────────────────────┘
                                                 │
                                    ┌─────────────────────────────┐
                                    │ Re-sample pulse wave signal based on samples│——112
                                    │ and time stamps using a cubic spline interpolation│
                                    │ in order to obtain re-sampled pulse wave signal│
                                    └─────────────────────────────┘
                                                 │
                                    ┌─────────────────────────────┐
                                    │ Filter re-sampled pulse wave signal to│——114
                                    │ eliminate noise and to compensate for drift│
                                    └─────────────────────────────┘
                                                 │
116'
┌─────────────────────────┐        ┌─────────────────────────────┐
│ Obtain reliability signal from│    │ Obtain original pulse wave signal│——116
│ accelerometer data       │        │ for processing               │
└─────────────────────────┘        └─────────────────────────────┘

118 —( END )
```

FIG.1B

FIG.1A

FIG.2

FIG.3

FIG.4

FIG.5

FIG.6

FIG.7A

FIG.7B

Europäisches
Patentamt

European
Patent Office

Office européen
des brevets

# EUROPEAN SEARCH REPORT

Application Number

EP 17 17 1812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/018408 A1 (OUCHI KAZUSHIGE [JP] ET AL) 15 January 2009 (2009-01-15) | 1-3,5-11 | INV. A61B5/024 A61B5/00 A61B5/021 A61B5/08 A61B5/1455 |
| Y | * abstract * <br> * claims 1-14 * <br> * paragraphs [0003], [0007], [0008], [0009], [0028], [0030], [0031], [0032], [0033], [0034], [0036], [0047], [0050], [0052], [0067] * <br> * figures 1,8,14 * | 4,14 | |
| Y | US 2016/220188 A1 (CHON KI H [US] ET AL) 4 August 2016 (2016-08-04) <br> * paragraph [0029] - paragraph [0034] * | 4 | |
| Y | COHEN H ET AL: "Autonomic dysfunction in patients with fibromyalgia: Application of power spectral analysis of heart rate variability", <br> SEMINARS IN ARTHRITIS AND RHEUMAT, ELSEVIER, AMSTERDAM, NL, <br> vol. 29, no. 4, <br> 1 February 2000 (2000-02-01), pages 217-227, XP004680862, <br> ISSN: 0049-0172, DOI: 10.1016/S0049-0172(00)80010-4 <br> * table 6 * | 14 | |
| X | US 2016/361023 A1 (MARTIN RUSSEL ALLYN [US] ET AL) 15 December 2016 (2016-12-15) <br> * abstract * <br> * claims 1-30 * <br> * paragraph [0039] - paragraph [0041] * | 1,7-10, 12,13,15 | |

TECHNICAL FIELDS SEARCHED (IPC)

A61B

-/--

The present search report has been drawn up for all claims

| Place of search | Date of completion of the search | Examiner |
|---|---|---|
| The Hague | 31 October 2017 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

# EUROPEAN SEARCH REPORT

Application Number

EP 17 17 1812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Wan-Hua Lin ET AL: "Comparison of Heart Rate Variability from PPG with That from ECG"<br>In: "IFMBE proceedings (International Federation for Medical and Biological Engineering)",<br>1 January 2014 (2014-01-01), Springer, DE,<br>XP055420109,<br>ISSN: 1680-0737<br>vol. 42, pages 213-215, DOI:<br>10.1007/978-3-319-03005-0_54,<br>* page 214, paragraph 1 *<br>* page 214, paragraph 4 *<br>* figure 2 * | 13,15 | |
| A | WO 2014/020484 A2 (KONINKL PHILIPS NV [NL]) 6 February 2014 (2014-02-06)<br>* claims 1-20 *<br>* figure 3 *<br>* page 9, line 5 - line 9 * | 6 | |
| A | SAMMITO STEFAN ET AL: "Reference values for time- and frequency-domain heart rate variability measures",<br>HEART RHYTHM, ELSEVIER, US,<br>vol. 13, no. 6,<br>12 February 2016 (2016-02-12), pages 1309-1316, XP029546627,<br>ISSN: 1547-5271, DOI:<br>10.1016/J.HRTHM.2016.02.006<br>* tables 2,3 * | 14 | TECHNICAL FIELDS SEARCHED (IPC) |

-/--

The present search report has been drawn up for all claims

| Place of search | Date of completion of the search | Examiner |
|---|---|---|
| The Hague | 31 October 2017 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 17 17 1812

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S. SAMMITO ET AL: "Guideline for the application of heart rate and heart rate variability in occupational medicine and occupational science", ASU INTERNATIONAL, vol. 2015, no. 06, 9 June 2015 (2015-06-09), XP055420114, DOI: 10.17147/ASUI.2015-06-09-03 * table 2 * | 13,15 | |
| A | M. MALIK ET AL: "Heart rate variability: Standards of measurement, physiological interpretation, and clinical use", EUROPEAN HEART JOURNAL, vol. 17, no. 3, 1 March 1996 (1996-03-01), pages 354-381, XP055420117, GB ISSN: 0195-668X, DOI: 10.1093/oxfordjournals.eurheartj.a014868 * page 364, paragraph 3 * | 13,15 | |

TECHNICAL FIELDS
SEARCHED (IPC)

The present search report has been drawn up for all claims

| Place of search | Date of completion of the search | Examiner |
|---|---|---|
| The Hague | 31 October 2017 | Delval, Christophe |

EPO FORM 1503 03.82 (P04C01)

## ANNEX TO THE EUROPEAN SEARCH REPORT
## ON EUROPEAN PATENT APPLICATION NO.

EP 17 17 1812

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2017

| Patent document cited in search report | | | Publication date | Patent family member(s) | | | Publication date |
|---|---|---|---|---|---|---|---|
| US 2009018408 | A1 | | 15-01-2009 | JP | 5060186 | B2 | 31-10-2012 |
| | | | | JP | 2009011585 | A | 22-01-2009 |
| | | | | US | 2009018408 | A1 | 15-01-2009 |
| US 2016220188 | A1 | | 04-08-2016 | US | 2016220188 | A1 | 04-08-2016 |
| | | | | WO | 2016123484 | A1 | 04-08-2016 |
| US 2016361023 | A1 | | 15-12-2016 | NONE | | | |
| WO 2014020484 | A2 | | 06-02-2014 | CN | 104684459 | A | 03-06-2015 |
| | | | | EP | 2879570 | A2 | 10-06-2015 |
| | | | | JP | 2015523182 | A | 13-08-2015 |
| | | | | RU | 2015106677 | A | 20-09-2016 |
| | | | | US | 2015190096 | A1 | 09-07-2015 |
| | | | | WO | 2014020484 | A2 | 06-02-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **W. KARLEN ; K. KOBAYASHI ; J. M. ANSERMINO ; G. A. DUMONT.** Photoplethysmogram signal quality estimation using repeated Gaussian filters and cross-correlation. *Physiol. Meas.,* 2012, vol. 33, 1617-1629

- Optimal Signal Quality Index for Photoplethysmogram Signals. *Mohamed Elgendi, Bioengineering,* 21 March 2016